# EUROPEAN PATENT APPLICATION

(11) **EP 2 505 630 A1**
(43) Date of publication of application: **03.10.2012**
(21) Application number: 10833098.6
(22) Date of filing: 16.11.2010
(51) Int. Cl.: C09K 3/00, A61K 8/06, A61K 8/25, A61K 8/46, A61K 8/49, A61Q 1/10, A61Q 1/12, A61Q 3/02, A61Q 17/04, A61Q 19/00

(54) **CLAY MINERAL THAT INCLUDES ULTRAVIOLET ABSORBER AND COSMETICS CONTAINING SAME**

(30) Priority: 24.11.2009 JP 2009266158
(71) Applicant: Shiseido Company, Ltd., Chuo-ku Tokyo 104-8010 (JP)
(72) Inventor: HATA, Hideo, Yokohama-shi Kanagawa 224-8558 (JP)
(74) Representative: Henkel, Breuer & Partner
(86) International application number: PCT/JP2010/070327
(87) International publication number: WO 2011/065251

(57) **Abstract**

The present invention provides an ultraviolet absorber-including clay mineral, which allows an ultraviolet absorber to be blended, as a powder component, into cosmetic preparations, can effectively absorb a wide range of ultraviolet light, and has excellent inclusion amount and inclusion strength; and cosmetics containing the same. The ultraviolet absorber-including clay mineral is characterized in that a polybase and an anionic ultraviolet absorber are intercalated between the layers of a water-swellable clay mineral. The polybase is preferably a polymer having a nitrogen-containing group that can become a cation. The anionic ultraviolet absorber is preferably one or more selected from 2-phenylbenzimidazole-5-sulfonic acid, 2-hydroxy-4-methoxybenzophenone-5-sulfonic acid, and their sodium salts.

## Description

### RELATED APPLICATIONS

This application claims the priority of Japanese Patent Application No. 2009-266158 filed on November 24, 2009, which is incorporated herein by reference.

### FIELD OF THE INVENTION

The present invention relates to an ultraviolet absorber-including clay mineral and cosmetics containing the same, and in particular, relates to the improvement in the ultraviolet protection ability of the ultraviolet absorber-including clay mineral, and the inclusion amount and the inclusion strength of the included anionic ultraviolet absorber therein.

### BACKGROUND ART

It has been known that a water-swellable clay mineral forms a composite with various organic compounds. That is, a water-swellable clay mineral has a structure in which plate crystals are layered and cations and water molecules are present between the layers; therefore, a composite can be easily obtained by including (intercalating) other molecules through the exchange of the cations or the water molecules present between the layers. Thus, it is possible to provide various functions to a water-swellable clay mineral by forming a composite with various functional molecules.

It has been reported that an ultraviolet absorber is included (intercalated) into a water-swellable clay mineral (Patent Literatures 1 to 3). In these, however, the interlayer interaction between the ultraviolet absorber and the clay mineral is of a non-covalent-bond type and relatively weak, and therefore the desorption has occurred in a dilute solution when in an equilibrium state.

On the other hand, it has also been reported that an anionic ultraviolet absorber is intercalated into a layered double hydroxide, of which the layer charge is positive (Patent Literature 4). However, the interlayer of the layered double hydroxide is easily ionized with carbonate ions due to carbon dioxide in the air. The interlayer carbonate ions must be substituted with nitrate ions for the subsequent intercalation reaction and thus, there is a disadvantage in that a large amount of acid needs to be used.

Furthermore, it has been reported that a polybase or a polycation is intercalated between the layers of a clay mineral to convert the electric charge between the layers from negative to positive, and the obtained composite is used for the intercalation of an acid dye (Patent Literature 5 and Non-patent Literature 1).

### PRIOR LITERATURES

### PATENT LITERATURES

Patent Literature 1: Japanese Unexamined Patent Publication No. S60-81124
Patent Literature 2: Japanese Unexamined Patent Publication No. S62-181213
Patent Literature 3: Japanese Unexamined Patent Publication No. H8-239648
Patent Literature 4: Japanese Unexamined Patent Publication No. 2008-1774
Patent Literature 5: Japanese Unexamined Patent Publication No. 2004-331878

### NON-PATENT LITERATURES

Non-patent Literature 1: Chemistry of Materials, 19, 79 (2007)

### DISCLOSURE OF THE INVENTION

### PROBLEM TO BE SOLVED BY THE INVENTION

However, one in which the included target material is an acid dye is hardly any ultraviolet-absorbing capability and thus has never been used as an ultraviolet absorber. In addition, many of the conventionally used ultraviolet absorbers are liquid, and it has been difficult to blend them into a cosmetic preparation as a powder component.
The present invention was made in view of the above-described problems of the conventional art. The object is to provide an ultraviolet absorber-including clay mineral that allows an ultraviolet absorber to be blended, as a powder component, into cosmetic preparations and can effectively absorb a wide range of ultraviolet light. Another object is to provide an ultraviolet absorber-including clay mineral having excellent inclusion amount and inclusion strength, and to provide cosmetics containing the same.

### MEANS TO SOLVE THE PROBLEM

In order to achieve the objects, the present inventors have diligently studied; as a result, the present inventors have found that an ultraviolet absorber-including clay mineral, which can achieve the objects, can be obtained by intercalating a polybase between the layers of a water-swellable clay mineral having an anionic layer charge to obtain cationic sites between the layers of the polybase-inorganic layered nanosheet composite, and by additionally intercalating an anionic water-soluble organic ultraviolet absorber between the layers.

That is, the ultraviolet absorber-including clay mineral of the present invention is characterized in that a polybase and an anionic ultraviolet absorber are intercalated between the layers of a water-swellable clay mineral.
In the ultraviolet absorber-including clay mineral of the present invention, it is preferable that the polybase is a polymer having a nitrogen-containing group that can become a cation.
In the ultraviolet absorber-including clay mineral of the present invention, it is preferable that the anionic ultraviolet absorber is one or more selected from the group consisting of 2-phenylbenzimidazole-5-sulfonic acid, 2-hydroxy-4-methoxybenzophenone-5-sulfonic acid, and their sodium salts.
The cosmetic of the present invention characteristically is characterized by comprising the ultraviolet absorber-including clay mineral.

The method for producing an ultraviolet absorber-including clay mineral of the present invention is characterized by comprising steps of:
preparing a polybase-including clay mineral by contacting a polybase with a water-swellable clay mineral in a water phase to intercalate the polybase between the layers of the water-swellable clay mineral; and
preparing the ultraviolet absorber-including clay mineral by mixing the polybase-including clay mineral with an anionic ultraviolet absorber in a water phase to additionally intercalate the anionic ultraviolet absorber between the layers.

### EFFECT OF THE INVENTION

According to the present invention, a polybase-including clay mineral, wherein a polybase is intercalated between the layers of a water-swellable clay mineral, is mixed with an anionic ultraviolet absorber, thereby obtaining an ultraviolet absorber-including clay mineral, wherein the anionic ultraviolet absorber is intercalated between the layers. With the use of this ultraviolet absorber-including clay mineral, it is possible to blend a liquid ultraviolet absorber into a cosmetic preparation in the form of powder. In addition, a wide range of ultraviolet light can be effectively absorbed because of the inclusion of the anionic ultraviolet absorber. Furthermore, an ultraviolet absorber-including clay mineral in which a large amount of the anionic ultraviolet absorber is included with excellent inclusion strength, and cosmetics containing the same can be obtained.

### BRIEF DESCRIPTION OF THE DRAWINGS

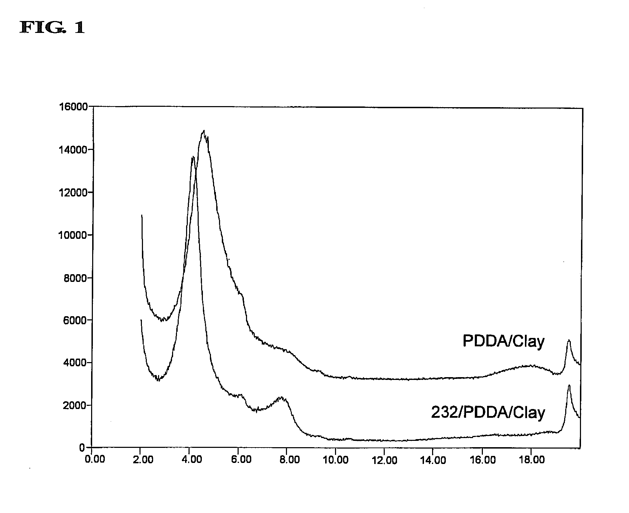

Fig. 1 shows the interlayer distance, of the ultraviolet absorber-including clay mineral of the present invention (Example 1), associated with the intercalation of a polybase (PDDA) and an anionic ultraviolet absorber (2-phenylbenzimidazole-5-sulfonic acid) between the layers of a water-swellable clay mineral (Clay), and it also shows the interlayer distance associated with the intercalation of a polybase (PDDA) between the layers of a water-swellable clay mineral (Clay).

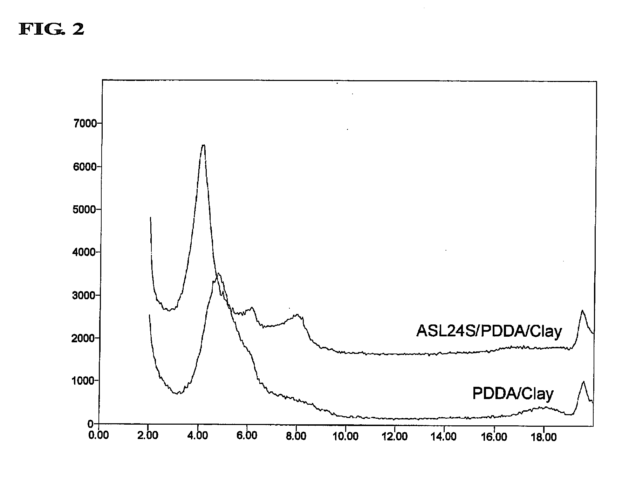

Fig. 2 shows that the interlayer distance, of the ultraviolet absorber-including clay mineral of the present invention (Example 1), associated with the intercalation of a polybase (PDDA) and an anionic ultraviolet absorber (sodium hydroxybenzophenonesulfonate) between the layers of a water-swellable clay mineral (Clay), and it also shows the interlayer distance associated with the intercalation of a polybase (PDDA) between the layers of a water-swellable clay mineral (Clay).

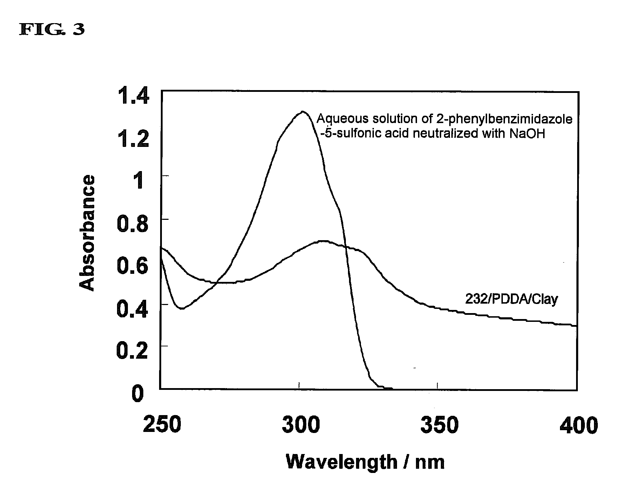

Fig. 3 shows the ultraviolet absorption spectrum of the ultraviolet absorber-including clay mineral of the present invention (Example 1) and that of the ultraviolet absorber aqueous solution.

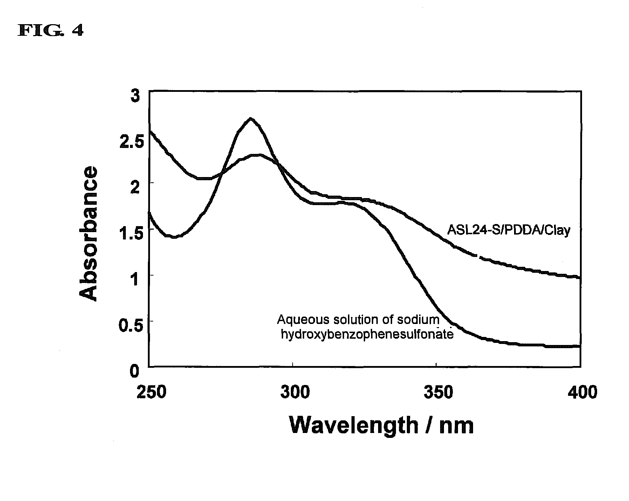

Fig. 4 shows the ultraviolet absorption spectrum of the ultraviolet absorber-including clay mineral of the present invention (Example 2) and that of the ultraviolet absorber aqueous solution.

### MODE FOR CARRYING OUT THE INVENTION

The ultraviolet absorber-including clay mineral of the present invention is a composite obtained by preparing a polybase-including clay mineral, wherein a polybase is included between the layers of a water-swellable clay mineral, and allowing an anionic ultraviolet absorber having the electric charge opposite to that of the polybase to adsorb to or coat the polybase-including clay mineral by electrostatic interaction.
In the technology known in the past, an ultraviolet absorber was simply intercalated in the clay mineral. It has not been reported that, by considering the electric charge, a cationic charged polybase is included in an anionic charged clay mineral and an anionic charged ultraviolet absorber is further included therein: thus, it was achieved for the first time in the present invention.

### Ultraviolet Absorber-Including Clay Mineral

The ultraviolet absorber-including clay mineral of the present invention will be explained.
The ultraviolet absorber-including clay mineral is a water-swellable clay mineral wherein a polybase and an anionic ultraviolet absorber are intercalated between the layers of the water-swellable clay mineral.
A water-swellable clay mineral generally has a negative surface charge. An anionic ultraviolet absorber also has an anionic group such as an SO₃- group. Therefore, it is normally difficult to intercalate an anionic ultraviolet absorber between the layers of a water-swellable clay mineral by simply mixing the two in water because of electrostatic repulsion. Even when an anionic ultraviolet absorber is adsorbed on the surface of clay mineral, it is easily desorbed by washing etc..
By intercalating a polybase between the layers of a water-swellable clay mineral, however, an anionic ultraviolet absorber can also be easily intercalated between the layers. This is because the interlayer charge of the water-swellable clay mineral inverts (from negative to positive) owing to the polybase. The intercalated anionic ultraviolet absorber does not easily desorb even by washing.

As the polybase, a polymer having a plural number of cationizable nitrogen-containing groups (can be salt forms) such as quaternary ammonium groups, amines, imines, or pyridines in the molecule is suitably used. It is preferable that the polybase is in the form of a polycation at least when contacted with a water-swellable clay mineral in a water phase. In order to cationize a polybase, an acid such as hydrochloric acid can be used as necessary. From the viewpoint of production process, it is preferable that the polybase has a chain structure and be water-soluble.

Specific examples of polybases include polyethyleneimine; amines such as polyallylamine, polyvinylamine, polyvinylpyridine, and salts thereof; quaternary ammonium salts such as polydimethyldiallylammonium salt and polymethacrylamidepropyltrimethylammonium salt; and cationized polysaccharides such as cationized cellulose, cationized guar gum, cationized locust bean gum, cationized β-1,3-glucan, and cationized starch. They can be either a homopolymer or a copolymer. Examples of the salts include salts with a halogen (Cl, Br, etc.).
Among them, polydimethyldiallylammonium chloride (PDDA) is especially preferable because it is an ideal strong polybase with uniform electric charge and is not affected by pH. In addition, PDDA is preferable from the standpoint of safety because it is acceptable for cosmetics.

There are polybases with various molecular weights. In the present invention, the molecular weight is not limited in particular so far as the polybase can provide an electric charge that can allow sufficient intercalation of an anionic ultraviolet absorber into the clay mineral. However, if the molecular weight of a used polybase is large, it can be thought that a water-swellable clay mineral tends to easily aggregate when the polybase is intercalated between the layers of the water-swellable clay mineral. Therefore, the average molecular weight of the polybase is preferably 1 million or less, and more preferably 100 thousand or less.

A polybase in which the polymer chain is flexible and the distance between the basic sites or cationic sites is large is advantageous because it has a coiled conformation between the layers of the water-swellable clay mineral. Thus, polydiallyldimethylammonium, polyallylamine, or a copolymer thereof with acrylamide, which acts as a spacer, is desirable.
A polybase is normally used as an aqueous solution of a suitable concentration when intercalated in a water-swellable clay mineral.

As the anionic ultraviolet absorber in the present invention, any anionic ultraviolet absorbers can be used so far as they can be included between the layers by the electrostatic interaction with the polybase. Among them, those having a sulfonate group (-SO₃H) or a phenol group (-OH) are preferable. These anionic groups can be in the form of a dissociable salt generated with a base (e.g., potassium hydroxide, sodium hydroxide). The salt is preferably an alkali metal salt, and especially preferably a sodium salt. It is preferable that the anionic group is in the form of an anion at least when it is in contact with the polybase-including clay mineral in water.

As the sulfonic acid-type anionic ultraviolet absorber,
2-phenylbenzimidazole-5-sulfonic acid, 2-hydroxy-4-methoxybenzophenone-5-sulfonic acid (Benzophenone-4), monosodium 2-hydroxy-4-methoxybenzophenone-5-sulfonate (Benzophenone-5), and disodium
2,2'-dihydroxy-4,4'-dimethoxybenzophenone-3,3'-disulfonate (Benzophenone-9) are preferable.

As the phenol-type anionic ultraviolet absorber, 2,4-dihydroxybenzophenone (Benzophenone-1), 2,2'-4,4'-tetrahydroxybenzophenone (Benzophenone-2), 2-hydroxy-4-methoxybenzophenone(Benzophenone-3),
2,2'-dihydroxy-4,4'-dimethoxybenzophenone (Benzophenone-6),
5-chloro-2-hydroxybenzophenone (Benzophenone-7),
2,2'-dihydroxy-4-methoxybenzophenone (Benzophenone-8),
2-hydroxy-4-methoxy-4'-methylbenzophenone (Benzophenone-10),
2-hydroxy-4-octoxybenzophenone (Benzophenone-12), homomenthyl salicylate (homosalate), and 2-ethylhexyl salicylate (octyl salicylate) are preferable.

In the present invention, 2-phenylbenzimidazole-5-sulfonic acid or monosodium 2-hydroxy-4-methoxybenzophenone-5-sulfonate (Benzophenone-5) is especially preferable.

2-Phenylbenzimidazole-5-sulfonic acid is commercially available, for example, as "Neo Heliopan Hydro" (manufactured by Symrise) and "Eusolex 232"(manufactured by Merck), and these can be suitably used. Preferably, it is used as a dissociable salt form after neutralizing with a base such as sodium hydroxide.

Monosodium 2-hydroxy-4-methoxybenzophenone-5-sulfonate (Benzophenone-5) is commercially available, for example, as "ASL-24S", which can be suitably used.
An anionic ultraviolet absorber is normally used as an aqueous solution of a suitable concentration when intercalated in a water-swellable clay mineral.

The water-swellable clay mineral used in the present invention is not limited in particular, and the examples include layered silicate minerals of the smectite group. Specifically examples of the layered silicate minerals of the smectite group include montmorillonite, beidellite, nontronite, saponite, and hectorite. Either natural or synthetic clay minerals can be used in the present invention. Examples of commercial water-swellable clay minerals include Kunipia, Smecton (Kunimine Industries Co., Ltd.), Veegum (R.T. Vanderbilt Company, Inc.), Laponite (Laporte Industries Ltd.), and synthetic fluorotetrasilicic mica (manufactured by Co-op Chemical Co., Ltd. or manufactured by Topy Industries, Ltd.). In the present invention, one or more can be arbitrarily selected for use from these layered silicates of the smectite group.

The primary particle size of the water-swellable clay mineral is preferably 5 microns or less, and more preferably 1 micron or less.
Furthermore, a water-swellable clay mineral with a higher cation exchange capacity (CEC) is more desirable because it can include larger amounts of a polybase and an anionic ultraviolet absorber. Therefore, the water-swellable clay mineral with 60 meq/100 g of clay or higher is desirable. When a polybase or an anionic ultraviolet absorber is intercalated or when the obtained inclusion clay mineral is coated on a base powder having the opposite surface charge, a dispersion containing the clay mineral in water at about the concentration where the gelation does not take place is used.

In the ultraviolet absorber-including clay mineral of the present invention, the amount of the polybase is preferably in an amount equivalent to one time or more the cation exchange capacity (CEC) of the water-swellable clay mineral, and more preferably 1.5 times or more. If the amount of the polybase is small, the interlayer charge of the clay mineral will not become sufficiently positive, and an anionic ultraviolet absorber may not be sufficiently included in the clay mineral. The amount of a polybase to be included varies according to the CEC of the used water-swellable clay mineral and the size of the polybase. The amount of an ultraviolet absorber to be included is proportional to the amount of the included polybase. Therefore, the amount of the polybase in the ultraviolet absorber-including clay mineral is preferably close to the saturation adsorption amount.

The amount of the anionic ultraviolet absorber in the ultraviolet absorber-including clay mineral of the present invention can be suitably set according to the desired ultraviolet absorption effect. Normally, it is in an amount equivalent to 0.1 times or more the cation exchange capacity (CEC) of the water-swellable clay mineral, more preferably 0.5 times or more, and most preferably one time or more. If the amount of the anionic ultraviolet absorber is small, the ultraviolet absorption effect may not be satisfactory.

The preparation of an ultraviolet absorber-including clay mineral can be carried out by bringing a water-swellable clay mineral and a polybase in contact in water and further bringing an anionic ultraviolet absorber in contact therewith in water. For example, a water-swellable clay mineral is sufficiently dispersed in water at about the concentration where the gelation does not take place (for example, 1 to 5 mass %) to prepare a water suspension. To this, a polybase aqueous solution (for example, an aqueous solution containing a polybase in an amount equivalent to 1 to 10 times the CEC of the water-swellable clay mineral) is mixed, and then an anionic ultraviolet absorber aqueous solution (for example, an aqueous solution containing a polybase in an amount equivalent to 0.1 to 10 times the CEC of the water-swellable clay mineral) is further mixed thereto. The obtained mixture is, as necessary, subjected to solid-liquid separation (e.g., centrifugation), washing, drying, and pulverization to obtain the powder of the ultraviolet absorber-including clay mineral. Alternatively, an aqueous solution containing a polybase and an anionic ultraviolet absorber mixed therein can be added to the water suspension of a water-swellable clay mineral. However, it is preferable to add a polybase beforehand to achieve sufficient intercalation and then to add an anionic ultraviolet absorber.
The conditions, when a polybase or an anionic ultraviolet absorber is mixed with a water-swellable clay mineral, may be suitably determined depending upon the used raw materials or the like. Normally, it is stirred at room temperature for 1 to 24 hours.

The ultraviolet absorber-including clay mineral of the present invention can be subjected to a surface treatment by a publicly known method so far as the effect of the present invention is not undermined. Examples of treatments for hydrophobization include treatments with silicones, fluorinated alkyls, higher fatty acids, higher alcohols, fatty acid esters, metal soaps, amino acids, alkyl phosphates, cationic active agents, and dextrin fatty acid esters. Any other surface treatments can also be applied. The present invention also includes the ultraviolet absorber-including clay mineral that is such surface-treated.

The thus obtained ultraviolet absorber-including clay mineral of the present invention is a powder wherein a polybase and an anionic ultraviolet absorber are included in a water-swellable clay mineral. The ultraviolet absorber-including clay mineral of the present invention can be easily separated as a solid from the liquid phase by filtration or centrifugation and has a low aggregation property. Therefore, it is usable as a pigment as it is after simple pulverization. Furthermore, an anionic ultraviolet absorber is, due to the interposition of a polybase, strongly retained between the layers of the clay mineral in an intercalated state. Thus, the amount of included anionic ultraviolet absorber is larger than the conventional one, and the inclusion strength is very high.
It is also possible to make the ultraviolet absorber-including clay mineral obtained in the present invention to a powdery composite with a powder having the opposite surface charge, for example, by the method of Patent Literature 5 or other publicly known methods. As the "surface charge", for example, the zeta potential (ξ potential) measured by LEZA 60 (manufactured by Otsuka Electronics Co., Ltd.) using 0.1 M NaCl aqueous solution as the mobile-phase solvent can be adopted.

The cosmetics of the present invention can be produced by the ordinary method with the components normally used in cosmetics and pharmaceuticals within the range that the effect of the present invention is not impaired, in addition to the ultraviolet absorber-including clay mineral. Examples of oils include silicone oils such as dimethylpolysiloxane, cyclodimethylpolysiloxane, and methylphenylpolysiloxane; various hydrocarbon oils such as squalane, liquid paraffin, light isoparaffin, petrolatum, microcrystalline wax, ozokerite, and ceresin; higher fatty acids such as myristic acid, palmitic acid, stearic acid, oleic acid, isostearic acid, and behenic acid; higher alcohols such as cetyl alcohol, stearyl alcohol, oleyl alcohol, and batyl alcohol; esters such as cetyl 2-ethylhexanoate, 2-ethylhexyl palmitate, 2-octyldodecyl myristate, neopentylglycol 2-ethylhexanoate, glyceryl trioctanoate, 2-octyldodecyl oleate, isopropyl myristate, myristyl myristate, glyceryl triisostearate, glyceryl trioleate, glyceryl coconut oil fatty acid triester; fats and oils such as olive oil, avocado oil, jojoba oil, sunflower oil, safflower oil, camellia oil, shea butter, macadamia nut oil, mink oil, lanolin, lanolin acetate, liquid lanolin, and castor oil; waxes such as Japan wax; fluorinated oils such as perfluoropolyethers and perfluorocarbons; trimethylsiloxysilicate; silicone resins such as MDQ resin; and polymers such as polymeric silicone rubber and acrylic-modified silicone copolymer. Other examples of the components include powder components, coloring agents, film-forming agents, surfactants, ultraviolet absorbers, antioxidants, salts, preservatives, thickeners, moisturizers, perfumes, aqueous components, and agents such as vitamins, hormones, whitening agents, and antiphlogistics.

Examples of the cosmetics of the present invention are not limited in particular, and they include makeup cosmetics such as an oil-based foundation, emulsion foundation, powdery foundation, dual-use foundation, face powder, blusher, pressed powder, cheek color, lipstick, eyeliner, mascara, eyeshadow, and nail enamel; and skin care cosmetics such as a milky lotion, lotion, cream, sunscreen, and pre-makeup.

### EXAMPLES

Hereinafter, the present invention will be explained with reference to specific examples. However, the present invention is not limited by these examples. The blending quantity is expressed in mass % unless otherwise specified.

### Preparation Of Ultraviolet Absorber-Including Clay Mineral

### Example 1:

0.75 g of sodium fluorotetrasilicic mica (Somasif ME-100, manufactured by Co-op Chemical Co., Ltd., CEC: 120 mequv/100 g) was added to 74.25 g of water and sufficiently dispersed in the water. The dispersion was mixed with stirring at room temperature for 24 hours, to obtain a clay aqueous dispersion. To the clay aqueous dispersion, 25 g of PDDA aqueous solution containing polydiallyldimethylammonium chloride (PDDA) (manufactured by Aldrich, low molecular grade) in an amount equivalent to 5 times the CEC was added. After mixed with stirring at room temperature for 24 hours, the mixture was subjected to centrifugal separation (12000 rpm × 30 min) and two washings, and then re-dispersed into 75 g of water. To the obtained dispersion, 16.7 g of sodium hydroxide aqueous solution (10 N) containing 0.165 g of 2-phenylbenzimidazole-5-sulfonic acid, which was in an amount equivalent to one time the CEC, was added. After mixed with stirring at room temperature for 1 day, the mixture was subjected to centrifugal separation (12000 rpm × 30 min), three washings, drying (70°C), and pulverization, to obtain an ultraviolet absorber-including clay mineral (232/PDDA/Clay) as powder.

### Example 2:

According to a treatment similar to the Example 1 except that 16.7 g of sodium hydroxybenzophenonesulfonate aqueous solution containing 0.198 g of sodium hydroxybenzophenonesulfonate, which was in an amount equivalent to one time the CEC, was added instead of the sodium hydroxide aqueous solution containing 2-phenylbenzimidazole-5-sulfonic acid, an ultraviolet absorber-including clay mineral (ASL-24S/PDDA/Clay) was obtained as powder.

### Interlayer Distance

In order to investigate whether the polybase PDDA, 2-phenylbenzimidazole-5-sulfonic acid, and sodium hydroxybenzophenonesulfonate were intercalated between the layers of the water-swellable clay mineral, the peak showing the interlayer spacing was checked with the XRD analysis (JDX-3500, manufactured by JEOL Ltd.) and the interlayer distance was also measured. The PDDA/Clay powder (PDDA/Clay), which was obtained by sampling a part of the liquid mixture before the addition of the anionic ultraviolet absorber in Examples 1 and 2, was similarly measured. The results are shown in Figs. 1 and 2.

From Fig. 1, it was confirmed that the peak assigned to the d (001) plane shifted to the lower angle side after the inclusion of 2-phenylbenzimidazole-5-sulfonic acid. It was also confirmed that the d value increased from 19.7 Å (PDDA/Clay) to 22.0 Å (232/PDDA/Clay) before and after the intercalation of 2-phenylbenzimidazole-5-sulfonic acid, and thus 2-phenylbenzimidazole-5-sulfonic acid was intercalated between the layers.

Also, in the case where sodium hydroxybenzophenonesulfonate was used as the anionic ultraviolet absorber, it was found that the d value of the peak assigned to the (001) plane increased from 18.6 Å (PDDA/Clay) to 21.6 Å (ASL-24S/PDDA/Clay) before and after the intercalation of sodium hydroxybenzophenonesulfonate, and thus sodium hydroxybenzophenonesulfonate was intercalated between the layers.
In all cases, a change in the interlayer distance when washing was hardly observed. Thus, it was considered that both polybase PDDA and anionic ultraviolet absorber were strongly included between the layers of the water-swellable clay mineral.

### Elemental Analysis

For the obtained powder, a CHN elemental analysis was carried out with 2400 II CHNS/O analyzer (manufactured by Perkin Elmer Co., Ltd.). The results are shown in Table 1.

**TABLE 1**

| Sample | C%(n=2) |
|---|---|
| PDDA/Clay | 11.28 |
| 232/PDDA/Clay | 15.19 |
| ASL-24S/PDDA/Clay | 16.17 |

As seen from Table 1, the carbon content in the 232/PDDA/Clay and that in the ASL-24S/PDDA/Clay increased compared with that in the PDDA/Clay. The results supported the above XRD analysis results showing that the polybase PDDA and the anionic ultraviolet absorber were intercalated between the layers.

### Absorbance Measurement

The ultraviolet absorption spectrum of the 232/PDDA/Clay in Example 1 and that of a solution of Eusolex 232 (2-phenylbenzimidazole-5-sulfonic acid) neutralized with sodium hydroxide (solvent: water, concentration: 10 ppm, light path length: 1 cm) were measured with a spectrophotometer (V-560, manufactured by JASCO Corporation). The results are shown in Fig. 3.
Similarly, the ultraviolet absorption spectrum of ASL-24S/PDDA/Clay in Example 2 and that of an aqueous solution of ASL-24S (sodium hydroxybenzophenonesulfonate) were measured. The results are shown in Fig. 4.

As is clear from Fig. 3, the wavelength of the maximum absorption of the anionic ultraviolet absorber, 2-phenylbenzimidazole-5-sulfonic acid, was 301 nm; however, in the 232/PDDA/Clay, wherein 2-phenylbenzimidazole-5-sulfonic acid was included between the layers of the water-swellable clay mineral, the wavelength of the maximum absorption shifted to 311 nm. In addition, 2-phenylbenzimidazole-5-sulfonic acid alone exhibited no absorption capability around 340 nm; however, the 232/PDDA/Clay exhibited some absorption capability even up to 400 nm.

As is also clear from Fig. 4, the wavelength of the maximum absorption of the ASL-24S/PDDA/Clay, wherein the anionic ultraviolet absorber sodium hydroxybenzophenonesulfonate is included, shifted to 287 nm from that of sodium hydroxybenzophenonesulfonate alone, 284 nm. In addition, the ASL-24S/PDDA/Clay exhibited the better absorption capability around the longer wavelength of 350 to 400 nm.
From the above results, the ultraviolet absorber-including clay mineral of the present invention was recognized to exhibit an excellent ultraviolet-absorbing capability in a wide range.

### Cosmetics

In the following, representative blending examples are shown for the cosmetics containing the ultraviolet absorber-including clay mineral of the present invention. These cosmetics can exhibit an ultraviolet absorption effect in a wide ultraviolet range.

### Blending Example 1 Dual-use foundation

| | |
|---|---|
| Silicone-treated talc | to 100 |
| Silicone-treated sericite | 20 |
| Silicone-treated mica | 10 |
| Silicone-treated titanium oxide | 10 |
| Silicone-treated red iron oxide | 0.8 |
| Silicone-treated yellow iron oxide | 3 |
| Silicone-treated black iron oxide | 0.2 |
| Ultraviolet absorber-including clay mineral of the invention | 3 |
| Liquid paraffin | 4 |
| Petrolatum | 4 |
| Sorbitan sesquiisostearate | 0.8 |
| Preservative | q.s. |
| Antioxidant | q.s. |
| Perfume | q.s. |

### Preparation method:

Powder components and oil-phase components were mixed with a Henschel mixer. The mixture was pulverized with a pulverizer two times, packed in a container (a plastic inner tray), and then dry-pressed according to the ordinary method.

### Blending Example 2 Single-use foundation

| | |
|---|---|
| Talc | to 100 |
| Sericite | 20 |
| Mica | 10 |
| Titanium oxide | 10 |
| Red iron oxide | 0.8 |
| Yellow iron oxide | 3 |
| Black iron oxide | 0.2 |
| Ultraviolet absorber-including clay mineral of the invention | 3 |
| Liquid paraffin | 4 |
| Petrolatum | 4 |
| Sorbitan sesquiisooleate | 0.8 |
| Preservative | q.s. |
| Antioxidant | q.s. |
| Perfume | q.s. |

### Preparation method:

According to a similar manner to the Blending Example 1, the preparation was carried out.

### Blending Example 3 Face powder

| | |
|---|---|
| Mica | 10 |
| Talc | to 100 |
| Zinc oxide | 5 |
| Fine particle titanium oxide | 3 |
| Ultraviolet absorber-including clay mineral of the invention | 0.1 |
| Fluorphlogophite | 10 |
| Petrolatum | 1 |
| Squalane | 2 |
| Ester oil | 1 |
| Preservative | q.s. |
| Antioxidant | q.s. |
| Perfume | q.s. |

### Preparation method:

According to a similar manner to the Blending Example 1, the preparation was carried out.

### Blending Example 4 Pre-makeup lotion

### Oil phase part

| | |
|---|---|
| Decamethylcyclopentasiloxane | to 100 |
| Polyether-modified silicone | 3 |
| Silicone-treated ultraviolet absorber-including clay mineral of the invention | 3 |
| Trimethylsiloxysilicate | 2 |
| Water phase part | |
| 1,3-Butylene glycol | 5 |
| Dynamite glycerin | 2 |
| Preservative | 0.5 |
| Purified water | 30 |

### Preparation method:

The water phase part was added to the oil phase part heated at 70°C and the mixture was sufficiently emulsified with a emulsifier. Then, the obtained emulsion was cooled while being stirred, and when the temperature was 35°C or lower, the emulsion was poured into a container. After the radiational cooling, the intended pre-makeup lotion was obtained.

### Blending Example 5 Eyeshadow

| | |
|---|---|
| (1)Talc | balance |
| (2)Sericite | 7 |
| (3)Mica | 15 |
| (4)Spherical PMMA powder | 3 |
| (5)Ultraviolet absorber-including clay mineral of the invention | 10 |
| (6)Barium sulfate | 4 |
| (7)Iron oxide | 1.5 |
| (8)Squalane | 2 |
| (9)Dimethylpolysiloxane | 2 |
| (10)Sorbitan monooleate | 0.5 |
| (11)Preservative | q.s. |
| (12)Perfume | q.s. |

### Preparation method:

Powder components and oil components were mixed with a Henschel mixer. The mixture was pulverized with a pulverizer two times, packed in a container (a plastic inner tray), and then dry-pressed according to the ordinary method.

### Blending Example 6 Oil-based stick

| | |
|---|---|
| (1)Carnauba wax | 1 |
| (2)Candelilla wax | 2 |
| (3)Ceresin | 10 |
| (4)Squalane | balance |
| (5)Glyceryl triisooctanoate | 9 |
| (6)Glyceryl diisostearate | 13 |
| (7)Dimethylpolysiloxane | 5 |
| (viscosity: 90,000 mPa·s at 25°C) | |
| (8)Dimethylpolysiloxane | 5 |
| (viscosity: 1,000 mPa·s at 25°C) | |
| (9)Silicone resin | 8 |
| (10)Hydroxypropyl-β-cyclodextrin | 1 |
| (11)Chotesteryl macadamia nuts fatty acid ester | 3.5 |
| (12)Synthetic sodium magnesium silicate | 0.5 |
| (13)Hydrophobic silica | 0.5 |
| (14)Purified water | 2 |
| (15)Spherical silicone resin powder-coated mica | 3 |
| (16)Silicone-treated ultraviolet absorber-including clay mineral of the invention | 5 |
| (17)Barium sulfate | 3 |
| (18)Coloring material | q.s. |
| (19)Preservative | q.s. |
| (20)Perfume | q.s. |

### Preparation method:

Components 12 and 13 were dispersed in Component 11 heated at 60°C. Components 10 and 14 were uniformly dissolved, and then added to the dispersion. The mixture was sufficiently stirred, and then added to Components 1 to 9 that were dissolved with heating beforehand. The obtained mixture was sufficiently mixed, and Components 5 to 20 were added thereto. The mixture was dispersed with stirring and then filled in a container to obtain the oily stick.

### Blending Example 7 Cream

### Oil phase part

| | |
|---|---|
| (1)Decamethylcyclopentasiloxane | 10.5 |
| (2)Dimethylpolysiloxane (6CS/25°C) | 4.0 |
| (3)Stearyl alcohol | 1.5 |
| (4)Petrolatum | 5.0 |
| (5)Squalane | 1.0 |
| (6)Vitamin E acetate | 0.01 |
| (7)Ultraviolet absorber-including clay mineral of the invention | 5.0 |
| (8)Polyether-modified silicone | 2.0 |
| Water phase part | |
| (9)Preservative | 0.2 |
| (10)1,3-Butylene glycol | 17.0 |
| (11)Purified water | balance |

### Preparation method:

According to the ordinary method, the cream was prepared.

### Blending Example 8 Sunscreen lotion

### Oil phase part

| | |
|---|---|
| (1)Dimethylpolysiloxane (6CS/25°C) | 5.0 |
| (2)Dimethylpolysiloxane (1.5CS/25°C) | 13.0 |
| (3)Phenyl-modified methylphenylpolysiloxane | 3.0 |
| (4)Ultraviolet absorber-including clay mineral of the invention | 5.0 |
| (5)Polyether-modified silicone | 2.0 |
| Water phase part | |
| (6)Sodium chloride | 9.0 |
| (7)Perfume | 0.2 |
| (8)Preservative | 0.2 |
| (9)Ethanol | 5.0 |
| (10)Purified water | balance |

### Preparation method:

According to the ordinary method, the sunscreen lotion was prepared.

### Blending Example 9 Liquid emulsion foundation

### Oil phase part

| | |
|---|---|
| (1)Decamethylcyclopentasiloxane | balance |
| (2)Trimethylsiloxysilicate | 3.0 |
| (3)Dimethylpolysiloxane | 5.0 |
| (4)Dimethylpolysiloxane-polyoxyalkylene copolymer | 2.5 |
| (5)Sorbitan sesquiisostearate | 2.0 |

### Powder part

| | |
|---|---|
| (6)Silicone-treated talc | 5.0 |
| (7)Silicone-treated titanium dioxide | 5.0 |
| (8)Ultraviolet absorber-including clay mineral of the invention | 5.5 |
| (9)Silicone-treated nylon powder | 4.0 |
| (10)Silicone-treated color pigment | 2.0 |
| Water phase part | |
| (11)1,3-Butylene glycol | 3.0 |
| (12)Ethanol | 13.0 |
| (13)Purified water | 10.0 |

### Preparation method:

The oil phase part was heated at 70°C and stirred, and the powder part was added thereto.
The mixture was stirred and dispersed with a homomixer at 70°C, and then cooled to the room temperature. The water phase was added to the dispersion and then the mixture was emulsified with a homomixer to prepare the liquid foundation.

### Blending Example 10 Cream emulsion foundation

### Oil phase part

| | |
|---|---|
| (1)Decamethylcyclopentasiloxane | balance |
| (2)Trimethylsiloxysilicate | 3.0 |
| (3)Dimethylpolysiloxane | 5.0 |
| (4)Sorbitan sesquiisostearate | 2.0 |
| (5)Dimethylpolysiloxane-polyoxyalkylene copolymer | 3.5 |
| (6)Dimethylstearylammonium hectorite | 2.0 |
| Powder part | |
| (7)Silicone-treated talc | 5.0 |
| (8)Silicone-treated titanium dioxide | 5.0 |
| (9)Silicone-treated ultraviolet absorber-including clay mineral of the invention | 2.0 |
| (10)Silicone-treated nylon powder | 4.0 |
| Water phase part | |
| (11)1,3-Butylene glycol | 3.0 |
| (12)Ethanol | 20.0 |
| (13)Purified water | 20.0 |

### Preparation method:

According to the Blending Example 9, the cream foundation was prepared.

### Blending Example 11 Nail enamel

| | |
|---|---|
| Nitrocellulose HIG 1/2 second | 10 |
| Nitrocellulose HIG 1/4 second | 5 |
| Alkyd resin | 10 |
| Acetyl tributyl citrate | 5 |
| Ethyl acetate | 25 |
| n-Butyl acetate | to 100 |
| n-Butyl alcohol | 5 |
| Ultraviolet absorber-including clay mineral of the invention | 2 |

### Preparation method:

According to the ordinary method, the nail enamel was prepared.

### Blending Example 12 Lotion

| | |
|---|---|
| (1)Ion-exchanged water | balance |
| (2)Ethanol | 10 |
| (3)Dipropylene glycol | 10 |
| (4)PEG1500 | 5 |
| (5)POE (20) oleyl alcohol ether | 0.5 |
| (6)Methyl cellulose | 0.3 |
| (7)Preservative | 0.2 |
| (8)Chelating agent | 0.01 |
| (9)Perfume | q.s. |
| (10)Ultraviolet absorber-including clay mineral of the invention | 0.01 |

### Preparation method:

Components 3, 4, 6 and 8 were dispersed a part of Component 1, and then Component 5, 7 and 9, which were dissolved in Component 2, was added thereto. Component 10, which was dispersed in the remaining Component 1 and stirred until it became transparent, was added to the mixture for coloring. Then, the obtained mixture was filtrated to obtain the lotion.

### Blending Example 13 Milky lotion

### Oils

| | | |
|---|---|---|
| | Dimethicone | 5 |
| | Cyclomethicone | 5 |
| | Liquid paraffin | 5 |
| Moisturizing Agents | | |
| | Glycerin | 4 |
| | 1,3-Butylene glycol | 5 |
| Polymers | | |
| | Carboxyvinyl polymer | 0.1 |
| | Acrylic acid-methacrylic acid acrylic copolymer | 0.1 |
| Neutralizer | | |
| | Potassium hydroxide | q.s. |
| Ultraviolet absorber-including clay mineral of the invention | | 0.01 |
| Preservative | | q.s. |
| Antioxidant | | q.s. |
| Perfume | | q.s. |
| Ion-exchanged water | | balance |

### Preparation method:

Moisturizing agents, preservative and Ultraviolet absorber-including clay mineral of the invention, which were dissolved with heating, and polymers were added to a part of ion-exchanged water. The mixture was dissolved at the room temperature and the neutralizer was added thereto, thereby preparing a water phase. Oils, antioxidant and perfume were uniformly mixed at the room temperature and added to the water phase. The mixture was emulsified with a homomixer, and the emulsion was deaerated and filtrated to obtain the milky lotion.

## Claims

1. An ultraviolet absorber-including clay mineral, wherein a polybase and an anionic ultraviolet absorber are intercalated between the layers of a water-swellable clay mineral.

2. The ultraviolet absorber-including clay mineral according to claim 1, wherein the polybase is a polymer having a nitrogen-containing group that can become a cation.

3. The ultraviolet absorber-including clay mineral according to claim 1 or 2, wherein the anionic ultraviolet absorber is one or more selected from the group consisting of 2-phenylbenzimidazole-5-sulfonic acid, 2-hydroxy-4-methoxybenzophenone-5-sulfonic acid, and their sodium salts.

4. A cosmetic, comprising the ultraviolet absorber-including clay mineral according to any of claims 1 to 3.

5. A method for producing an ultraviolet absorber-including clay mineral, comprising steps of:
preparing a polybase-including clay mineral by contacting a polybase with a water-swellable clay mineral in a water phase to intercalate the polybase between the layers of the water-swellable clay mineral; and
preparing the ultraviolet absorber-including clay mineral by mixing the polybase-including clay mineral with an anionic ultraviolet absorber in a water phase to additionally intercalate the anionic ultraviolet absorber between the layers.
